(19) Europäisches Patentamt / European Patent Office / Office européen des brevets

(11) **EP 2 767 266 A1**

(12) **EUROPEAN PATENT APPLICATION**

(43) Date of publication:
20.08.2014 Bulletin 2014/34

(51) Int Cl.:
A61F 13/00 (2006.01)      D02J 1/18 (2006.01)
D04H 3/14 (2012.01)      A61F 13/02 (2006.01)

(21) Application number: 14160425.6

(22) Date of filing: 08.12.2006

(84) Designated Contracting States:
AT BE BG CH CY CZ DE DK EE ES FI FR GB GR HU IE IS IT LI LT LU LV MC NL PL PT RO SE SI SK TR

(30) Priority: 14.11.2006 US 559507

(62) Document number(s) of the earlier application(s) in accordance with Art. 76 EPC:
06846525.1 / 2 081 527

(71) Applicant: Celanese Acetate LLC
Dallas, TX 75234 (US)

(72) Inventors:
• Neron, Rene B.
Blacksburg, VA Virginia 24060 (US)

• Robertson, Raymond M.
Blacksburg, VA Virginia 24060 (US)
• Suthar, Jitendrakumar N.
Blacksburg, VA Virginia 24060 (US)
• Dehart, Gary E.
Peterstown, WV West Virginia 24693 (US)

(74) Representative: Braun, André jr.
Braunpat Braun Eder AG
Reussstrasse 22
4054 Basel (CH)

Remarks:
This application was filed on 18-03-2014 as a divisional application to the application mentioned under INID code 62.

(54) **Wound care product made from bulked filament tow**

(57) A wound care product is a nonwoven fabric made of filaments bulked from a tow and fixed into a 3-dimensional structure.

Fig. 3

EP 2 767 266 A1

**Description**

Field of the Invention

A wound care product is made from a bulked filament tow.

Background of the Invention

The use of nonwoven materials as wound dressings is disclosed in, for example, U.S. Patent Nos. 4,704,113; 4,741,941; 6,448,462; and 6,500,539, each of which are discussed in greater detail below.

In general, materials used as wound dressings need, among other things, the ability to remove fluid from the wound site (a transport phenomenon), to hold the removed fluid (an absorption phenomenon), and not to adhere (stick) to the wound.

Nonwoven fabric is a term of art that refers to a manufactured sheet, batting, webbing, or fabric that is held together by various methods. Those methods include, for example, fusion of fibers (e.g., thermal, ultrasonic, pressure, and the like), bonding of fibers (e.g., resins, solvents, adhesives, and the like), and mechanical entangling (e.g., needle-punching, entangling, and the like). The term is sometimes used broadly to cover other structures such as those held together by interlacing of yarns (stitch bonding) or those made from perforated or porous films. The term excludes woven, knitted, and tufted structures, paper, and felts made by wet milling processes. In its most common usage, the term includes fibrous structures made by such processes as dry, wet, or air laying (with or without one of the methods of holding the fibers together mentioned above), needle-punching, spunbond or meltblown processes, and hydroentangling (spunlacing). In the dry, wet, air laying, and hydroentangling (spunlacing) processes, staple fibers are used in the manufacture of the nonwoven fabric. In the spunbond and meltblown processes, molten polymer is extruded onto a moving belt; the fibers of these types of nonwovens may be filaments.

U.S. Patent No. 4,704,113 discloses a staple fiber, hydroentangled nonwoven used as a hospital dressing.

.U.S. Patent No. 4,741,941 discloses a meltblown or spunbonded nonwoven that can used as a hospital wipe.

U.S. Patent No. 6,448,462 discloses a spunbonded nonwoven used as a bandage material.

U.S. Patent No. 6,500,539 discloses a spunlaced (hydroentangled) nonwoven as a wound dressing.

While each of the foregoing has provided an advancement in the art of wound care, there is a need for better wound care products.

Summary of the Invention

A wound care product is a nonwoven fabric made of filaments bulked from a tow and fixed into a 3-dimensional structure.

Description of the Figures

For the purpose of illustrating the invention, there is shown in the drawings a form that is presently preferred; it being understood, however, that this invention is not limited to the precise arrangements and instrumentalities shown.

Figure 1 is a photograph showing a flat tow (right hand side) and a bulked tow (left hand side).

Figure 2 is a photograph illustrating two embodiments of the present invention (right hand side and center) along with a flat tow (left hand side).

Figure 3 is schematic illustration of the process for making the instant invention.

Figure 4 is a graph illustrating the effect of a plasticizer on the shape retention of a nonwoven fabric after wetting.

Figure 5 is a graph illustrating the effect of a plasticizer on the shape retention of a nonwoven fabric after wetting.

Description of the Invention

Wound care product, as used herein, refers to post operative absorbent dressings (or pads), wound pads for cushioning, Gamgee dressings, sponges (including ultra small examples often known as 'pledgets') for use externally or internally, bandages, patient underpads, gauzes for skin preparation/debridement, gauzes including narrow or 'ribbon gauze,' and laporotomy sponges for internal operating room (OR) uses. This material may also be used as a component or in its entirety in a wound dressing, a component or in its entirety in a bandage, a component or in its entirety in an eye dressing, a component or in its entirety in a nursing pad, a component or in its entirety in absorbent materials used in autopsy, a component or in its entirety in dental dressings, a component or in its entirety in veterinary dressings, or one of the other listed applications. This material may also be used as wadding for the packaging of medicine in bottles or jars.

Nonwoven fabric as used herein refers to randomly oriented filaments produced from a bulked tow, and excludes nonwoven fabrics made by dry, wet, or air laying processes, needle-punching, spunbond or meltblown processes, and hydroentangling (spunlacing).

Filament refers to continuous fiber, i.e., a fiber of infinite length when compared to its cross-sectional diameter.

Tow refers to a bundle of filaments without definite twist.

Bulked (or bulking) refers to a processing step whereby an flat tow is caused to swell, grow, expand, and/or increase in thickness, for example, perpendicular to both the machine direction (MD) and the cross machine direction (CD) of the tow. Bulking may be accomplished by use of an air jet.

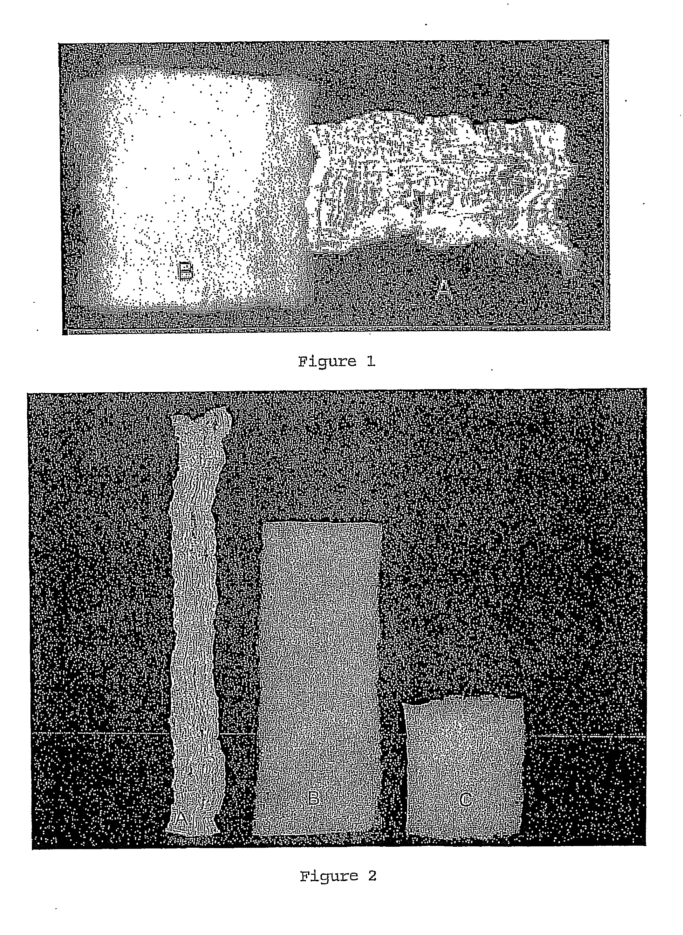

Referring to Figure 1, there is shown a tow A of filaments and a nonwoven B made from a bulked tow. Tow A is generally a flat structure with the filaments generally aligned with one another. The nonwoven B is bulked and has 3-dimensional structure with the filaments primarily oriented along the MD, with additional folding and deregistering of the filaments within the structure creating the additional bulk.

Referring to Figure 2, there is shown two embodiments B and C of the present invention of a nonwoven made from a bulked filament tow and having a fixed 3-dimensional structure. There is also shown a flat tow A. Each sample, A, B, & C, weighs 4.3 grams. Embodiments B & C demonstrate the bulk that can be achieved with the instant process.

The filaments may be made of any material that can be formed into filaments. Such materials may include melt spinnable polymers and solution spinnable polymers. Such material includes, but are not limited to: acrylics, cellulosics (e.g., regenerated celluloses (rayons), and cellulose esters), polyamide (e.g., nylons), polyesters (e.g., PET and PBT), polyolefins (e.g., PE, PB, PMP, PP), and mixtures thereof. In one embodiment, the filaments are made of cellulose acetate.

The filaments may have any size. The denier of an individual filament may range from 1-15 dpf (denier per filament). In one embodiment, the denier may range from 2-10 dpf. In another embodiment, the denier may range from 3-8 dpf.

The filaments may have any cross-sectional shapes. Such shapes include, but are not limited to: round, 'y,' 'x,' crenulated, dog bone, or combinations thereof.

The tow may include any number of filaments. The number of filaments may range in number from 2,500 to 25,000.

The tow may have any total denier. The total denier of the tow may be in the range of 2,500 to 125,000. In one embodiment, the total denier of the tow may range from 15,000 to 75,000. In another embodiment, the total denier of the tow may range from 20,000 to 40,000.

The tow may be crimped. Crimps may be in the range of 5-80 crimps per inch (2-32 crimps per cm). In one embodiment, the crimps may range from 25-35 crimps per inch (10-14 crimps per cm).

The tow may include a finish or may be finished. When a surface finish is applied, the finish may comprise about 0.3-5.0 wt% of the tow. In one embodiment, the finish comprises about 0.5-2.0 wt% of the tow.

The nonwoven fabric may have any physical dimension or any cross-sectional shape. In one embodiment, the nonwoven fabric may have the following physical dimensions: basis weight of 50-300 g/m$^2$; a width of 50-300 mm; and a thickness of 2 mm-5 cm. The cross-sectional shapes may include, for example, rectangular, square, round, or oval. In one embodiment, the cross-sectional shape may be rectangular.

The nonwoven fabric preferably has a fixed, 3-dimensional structure to facilitate, at least, transport of fluid away from the wound, absorbency capacity, and shape retention. The nonwoven fabric may be fixed by any means. The nonwoven fabric may be fixed by use of, for example: a binder (an adhesive-type material that cements the filaments to one another at filament contact points); a plasticizer (a material that softens the polymer of the filaments and allows the filaments to coalesce at filament contact points); and/or external energy source to form point bonds (such energy sources include, for example, thermal, pressure, and/or ultrasonic bonding techniques, which may or may not be facilitated by the use of bicomponent fibers incorporated into the nonwoven fabric).

The choice of the fixing technique may be dependent upon the polymer of the filament. For example, if the filament is a cellulose ester, e.g., cellulose acetate, a plasticizer may be used. Such plasticizers may be, for example, triacetin, triethylene glycol diacetate, glycol monoethyl ether acetate, and combinations thereof. In one embodiment, the plasticizer may be added to the nonwoven fabric in the range of 2-15 wt% of the nonwoven fabric. In another embodiment, the plasticizer may be added to the nonwoven in the range of 7-20 wt% of the nonwoven fabric.

The nonwoven fabric may also include the following, alone or in combination:

Radio-opaque detector mechanisms, such as threads or beads, that allows detection when used within the patient.

Radio frequency (RF) tags which could then be detected by an external counting or tracking system and that eliminate the need for manually counting surgical disposables before and after surgery.

Bar coding systems, such as tapes, which could then be detected by an external counting or tracking system, eliminating the need for manually counting surgical disposables before and after surgery.

Antimicrobial agents intended to slow or kill the growth of microbes and potentially reduce the occurrence of infection. Such agents are conventional and may include, but are not limited to, drugs, chemicals or the like. These agents may be added during filament spinning or with the agent used to fix the structure of the nonwoven fabric or added to the surface of the filaments in any known manner. Antimicrobial agents include, but are not limited to, antibacterial agents, antiviral agents, antifungal agents, and/or antiparisitic agents. Such agents may include, but are not limited to, silver ions, Chitosan, copper ions, and/or chlorinated phenoxy compounds.

The non-adherence properties of the nonwoven fabric may be improved by any known manner. For example, absorbent cellulose derivatives may be used. One absorbent cellulose derivative material is hydroxypropyl cellulose. This material may be added to the surface of the nonwoven fabric that is intended to be in contact with the wound surface. Alternatively, calcium alginate (derived from seaweed) may also be used. This material may be added in sheet or web form to a side of the nonwoven fabric that is intended for contact with the wound and readily dissolves when contacted by a saline solution prior to removal of the dressing from the wound. Calcium alginate is commercially available from Specialty Fibers and Materials, Ltd. In another embodiment, siloxanes may be added to the nonwoven fabric in any conventional manner.

Flexible absorbent binder (FAB) may be added to increase the absorbent capacity of the nonwoven fabric. FAB may be applied to the nonwoven fabric in any conventional manner. One such material is described in U.S. Patent No. 6,964,803, incorporated herein by reference.

The nonwoven fabric does not include any superabsorbent particles (SAP) that are commonly used in the manufacture of personal hygiene products/garments.

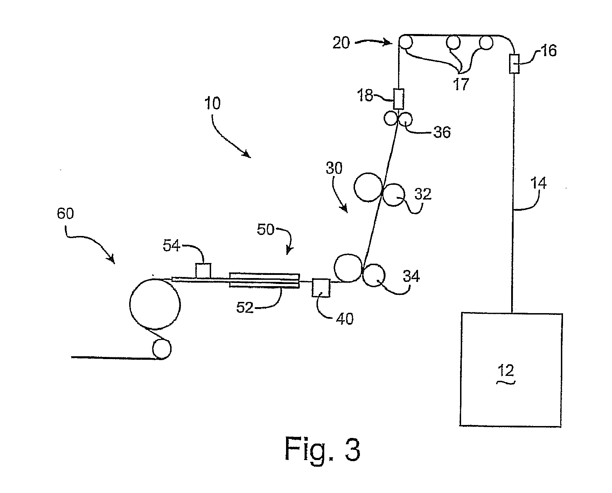

Referring to Figure 3, the manufacture of the instant wound dressing shall be described. The process 10 for making the wound care product generally comprises the steps of: bulking 50 the tow, and fixing 40 the 3-dimensional structure of the bulked tow. In the embodiment shown, bulking 50 the tow further includes spreading 20 the tow and deregistering 30 the tow.

Tow 14 may be pulled from a bale 12. The tow (or tow band) 14 may be spread 20 (i.e., increasing its width from the compressed state in the bale) by use of one or more banding jets 16, 18. During travel, the tow 14 may be guided by one or more guides 17. Additionally, multiple tows may be combined by feeding several tow bands together. In this way, the nonwoven may include differing fibers. Differing fibers may include, but is not limited to, fibers of differing sizes, fibers made of differing materials, fibers having differing additives or surface coatings, fibers of differing chemical, medical, and physical properties, and combinations thereof. With this flexibility, nonwovens with varying functions may be produced. In one specific example of the foregoing, calcium alginate fibers (which, for example, have beneficial gelling properties desired for contact with a wound surface) may be readily combined with other fibers (e.g., those mentioned above) to form a wound care product.

The spread tow is then deregistered 30 in deregistering apparatus that may consist of at least two pairs of driven rollers 32, 34. These driven rollers turn at different speeds. In one embodiment, rollers 34 turning faster than rollers 32. In one embodiment (not shown in the figure), one roller of each pair is grooved or threaded and the mate is smooth faced. Additionally, a pair of pretension rollers 36 may be used to facilitate deregistration of the filaments of the tow band.

Fixing the 3-dimensional structure of the bulked tow may be accomplished before, during, or after the tow is bulked.

In one embodiment, a plasticizer is added 40 to the deregistered tow prior to bulking to facilitate fixing of the 3-dimensional structure of the nonwoven fabric. The plasticizer may be added in any conventional manner. Application of the plasticizer may be by brushing, spraying, pads, wicks, or other types of plasticizer applicators. Further, the plasticizer may be applied to one or more sides of the tow/bulked tow. Optionally, when the plasticizer method of fixing is used, setting of the fixing may be sped up, i.e., reducing the set time. Speeding up the set may be accomplished in any conventional manner. One such manner may be by the injection of live steam into the bulked tow. The injection of steam may be further aided by a pair of nip rollers which additionally serve to control the thickness and density of the nonwoven fabric. Alternatively, a pair of heated godet rollers may be used to set the fix. These heated godet rollers contact the bulked tow and not only help set the 3-dimensional structure of the tow, but also control the thickness and density of the nonwoven fabric.

In another embodiment, fixing of the 3-dimensional structure may be accomplished after the tow is bulked. In this latter embodiment, the binder and/or the use of the external energy source are applied, in any conventional manner, after the tow has been bulked.

The deregistered tow is bulked 50 in any conventional manner. In one embodiment, the tow is bulked with an air jet 52. Such air jets 52 are known. See, for example, U.S. Patent Nos. 5,331,976 and 6,253,431, incorporated herein by reference. After bulking and before fixing, it may be necessary to carry the bulked tow because the bulked tow has little to no machine direction (MD) strength. For example, the bulked tow may be carried on: a discrete material (e.g., a tissue) or moving belt or a rotating drum (which may or may not be vacuum assisted). The tissue may be subsequently discarded or the tissue may be incorporated into a subsequent product based upon the nonwoven fabric. Additionally, the tissue may sandwich the bulked tow. By sandwiching the tow, the bulked tow would have the same characteristic on both sides. Tissue, as used here, includes, but is not limited to: tissue, woven fabric, knitted fabric, other nonwoven, same nonwoven, film or the like.

Optionally, a speed controller 54 may be used to control/regulate the basis weight of the nonwoven. Alternately, the basis weight of the nonwoven may be controlled by an additional pair of driven rollers (e.g., nip rollers) located immediately after the air jet.

After the bulked tow is fixed, it is ready for subsequent processing 60. Subsequent processing may include, but is not limited to: wind-up; addition of other material or components; sterilization; cutting to shape; packaging; subsequent bonding (e.g., external energy source or adhesives); calendaring; and combinations thereof. The instant nonwoven fabric may also be joined to one or more other substrates. Such substrates include, but are not limited to, films, meshes, nonwovens, or fabrics (woven or knitted). Non-limiting examples of the forgoing include; barrier films to reduce or prevent strikethrough of exudates from the dressing; scrims to provide additional strength to the nonwoven in the machine direction, cross machine direction, or both; and materials that provide additional tactile or aesthetic benefits to the final product.

Additional operating parameters of the foregoing process may be obtained from the relevant portions of U.S. Patent Nos. 6,253,431; 6,543,106; 7,059,027; and U.S. patent applications Serial Nos. 10/672,036; 10/672,109; 10/672,519; 10/672,673; 10/672,674, all filed September 26, 2003, and each of which is incorporated herein by reference.

The instant invention also includes a nonwoven as described above, but for uses other than wound care products.

Examples

The foregoing invention may be further illustrated with regard to the following non-limiting examples.

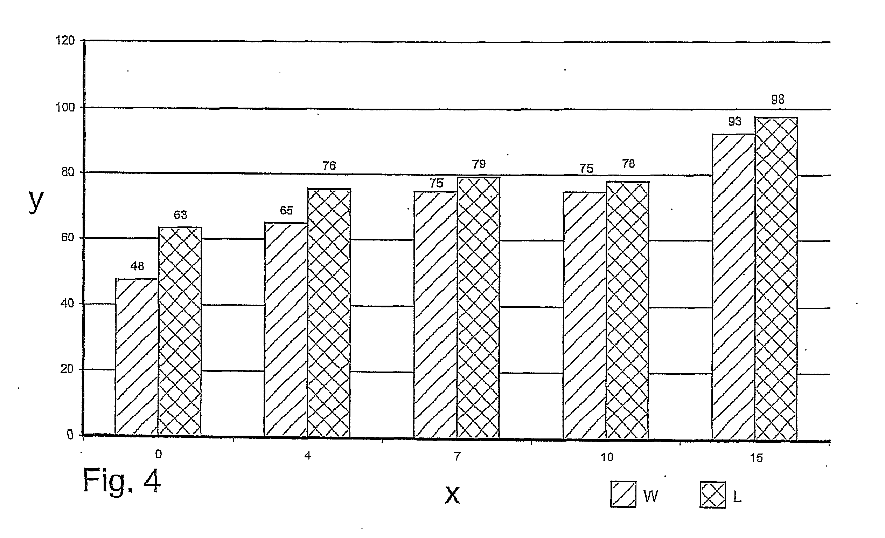

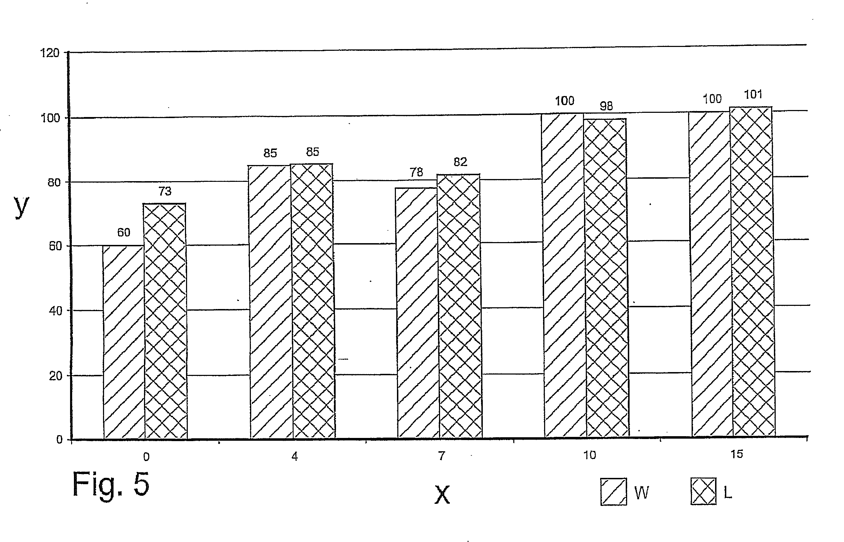

In the Figures 4 and 5, the shape retention properties of the subject nonwoven are illustrated. The X-axis illustrates the wt% of plasticizer per the total nonwoven weight and the y-axis illustrates the % of the original dimension. W represents the width and L represents the length of the material tested. Both samples were made according to the foregoing process with 110mm air jet disclosed in U.S. Patent No. 6,253,431, a basis weigh of 80 g/m$^2$, a line speed of 50 m/minute, and a bulking ratio of 1.5 (Bulking ratio refers to the linear speed of the tow across the second pair of rollers (in Figure 3, roller pair 34) relative to the linear speed of the tow across the first pair of rollers (32), and expressed as a ratio). The sample illustrated in Figure 4 was made of cellulose acetate (2.5 dpf, 30,000 total denier) and triacetin was used as plasticizer. The sample illustrated in Figure 5 was made of cellulose acetate (7.3 dpf, 33,000 total denier) and triacetin was used as plasticizer. The test was performed by placing a 10 cm x 41 cm sample in de-ionized water for 20 minutes, hanging the wetted sample vertically for 2 minutes and allowed to drip, the sample was then allowed to air dry, and the resulting dimensions were recorded. The shape retention values were calculated according to the following formula:

$$\texttt{Shape retention (\%) = [(original-post wetting)/original]*100.}$$

The present invention may be embodied in other forms without departing from the spirit and the essential attributes thereof, and, accordingly, reference should be made to the appended claims, rather than to the foregoing specification, as indicated the scope of the invention.

**Claims**

1. A wound care product comprising;
   a nonwoven fabric made of filaments bulked from a tow and being fixed into a 3-dimensional structure, and
   a material selected from the group consisting of radio-opaque detector, radio frequency tag, bar code tag, antimicrobial agent, flexible absorbent binder, and combinations thereof.

2. The wound care product of claim 1 wherein said nonwoven fabric having a basis weight in the range of 50-300 g/m$^2$.

3. The wound care product of claim 1 wherein said nonwoven fabric having a thickness in the range of 2 mm-5 cm.

4. The wound care product of claim 1 wherein said filaments being a thermoplastic material.

5. The wound care product of claim 4 wherein said thermoplastic material being acrylics, cellulosics, polyamides, polyolefins, polyesters, co-polymers thereof, and mixtures thereof.

6. The wound care product of claim 5 wherein said thermoplastic material being cellulose acetate.

7. The wound care product of claim 1 wherein said filaments comprising two or more differing filaments.

8. The wound care product of claim 1 further comprising a binder.

9. The wound care product of claim 8 wherein said binder comprising 4-20% by weight of said fabric.

10. The wound care product of claim 8 wherein said binder being selected from the group consisting of glycerol derivatives, cyclohexanone, benzl alcohol, and combinations thereof.

11. The wound care product of claim 10 wherein glycerol derivatives include glyceryl triacetate (triacetin), triethylglycerol diacetate, and glycol monoethyl ether acetate.

12. The wound care product of claim 1 further comprising point bonds.

13. The wound care product of claim 12 wherein said point bonds being selected from the group of thermal bonds, ultrasonic bonds, pressure bonds, and combinations thereof.

14. The wound care product of claim 1 wherein said fabric not including any superabsorbent particles incorporated therein.

15. A method of making a wound care product comprising the steps of:

   bulking a filament tow into a nonwoven fabric, and
   fixing the filaments into a 3-dimensional structure.

16. The method of claim 15 wherein said nonwoven fabric having a basis weight in the range of 50-300 $g/m^2$.

17. The method of claim 15 wherein bulking further comprising opening the tow with an air jet.

18. The method of claim 15 wherein fixing the filaments further comprising applying a binder to the nonwoven.

19. The method of claim 15 wherein fixing the filaments further comprising bonding the filaments of the nonwoven with an external energy source.

Figure 1

Figure 2

Fig. 3

Fig. 4

Fig. 5

EP 2 767 266 A1

## EUROPEAN SEARCH REPORT

**Application Number**

EP 14 16 0425

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 195 623 A (KANBAR MAURICE SHAMA [US]; SUDARSKY RAYMOND DAVID [US]) 17 June 1970 (1970-06-17) * page 1, line 53 - page 2, line 4 * * page 2, line 17 - line 60 * ----- | 1-5, 14-17 | INV. A61F13/00 D02J1/18 D04H3/14 A61F13/02 |
| X | GB 2 394 232 A (LOHMANN GMBH & CO KG [DE]) 21 April 2004 (2004-04-21) | 15-17,19 | |
| Y | * page 1, line 18 - page 2, line 22 * ----- | 1-19 | |
| Y | US 5 041 103 A (RUPINSKAS VYTAUTAS R [US]) 20 August 1991 (1991-08-20) * column 2, line 41 - line 66 * ----- | 1-19 | |

| TECHNICAL FIELDS SEARCHED (IPC) |
|---|
| D04H A61F D02G D02J |

The present search report has been drawn up for all claims

| Place of search | Date of completion of the search | Examiner |
|---|---|---|
| The Hague | 1 July 2014 | Van Beurden-Hopkins |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 16 0425

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2014

| Patent document cited in search report | | Publication date | Patent family member(s) | | Publication date |
|---|---|---|---|---|---|
| GB 1195623 | A | 17-06-1970 | NONE | | |
| GB 2394232 | A | 21-04-2004 | AU | 2003271900 A1 | 04-05-2004 |
| | | | GB | 2394232 A | 21-04-2004 |
| | | | WO | 2004035902 A1 | 29-04-2004 |
| US 5041103 | A | 20-08-1991 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4704113 A
- US 4741941 A
- US 6448462 A
- US 6500539 A
- US 6448462 B
- US 6500539 B
- US 6964803 B
- US 5331976 A
- US 6253431 A

- US 6253431 B
- US 6543106 B
- US 7059027 B
- US 672036 A
- US 10672109 A
- US 10672519 A
- US 10672673 A
- US 10672674 A